(19) **Europäisches Patentamt European Patent Office Office européen des brevets**

(11) **EP 1 525 305 B1**

(12) **EUROPEAN PATENT SPECIFICATION**

(45) Date of publication and mention of the grant of the patent:
**23.06.2010 Bulletin 2010/25**

(21) Application number: **03784438.8**

(22) Date of filing: **25.07.2003**

(51) Int Cl.:
*C12N 9/12* (2006.01)      *C12P 19/34* (2006.01)
*C12P 21/02* (2006.01)

(86) International application number:
**PCT/IB2003/003936**

(87) International publication number:
**WO 2004/015059 (19.02.2004 Gazette 2004/08)**

(54) **METHODS AND COMPOSITIONS FOR IN VITRO SYNTHESIS OF PROTEINS IN A CELL-FREE SYSTEM ENRICHED WITH ATP-SULFURYLASE**

METHODEN UND ZUSAMMENSTELLUNGEN FÜR DIE IN-VITRO SYNTHESE VON PROTEINEN IN EINEM ZELLFREIEN SYSTEM ANGEREICHERT MIT ATP-SULFURYLASE

PROCEDES ET COMPOSITIONS DESTINES A LA SYNTHESE IN VITRO DE PROTEINES DANS UN SYSTEME ACELLULAIRE ENRICHI EN ATP SULFURILASE

(84) Designated Contracting States:
**AT BE BG CH CY CZ DE DK EE ES FI FR GB GR HU IE IT LI LU MC NL PT RO SE SI SK TR**

(30) Priority: **02.08.2002 EP 02291959**

(43) Date of publication of application:
**27.04.2005 Bulletin 2005/17**

(73) Proprietor: **Proteus**
**30000 Nîmes (FR)**

(72) Inventors:
• **RYABOVA, Lyubov**
**Bât. B,Appt 206,**
**F-30900 Nîmes (FR)**

• **MASSON, Jean-Michel**
**F-31400 Toulouse (FR)**

(74) Representative: **Novagraaf Technologies**
**122, rue Edouard Vaillant**
**92593 Levallois-Perret Cedex (FR)**

(56) References cited:
**WO-A-98/22615**

• **KIM DONG-MYUNG ET AL: "Regeneration of adenosine triphosphate from glycolytic intermediates for cell-free protein synthesis."** BIOTECHNOLOGY AND BIOENGINEERING, vol. 74, no. 4, 20 August 2001 (2001-08-20), pages 309-316, XP002227479 ISSN: 0006-3592 cited in the application

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention pertains to the field of methods and compositions that enhance *in vitro* synthesis of proteins in a cell-free system.

**Background.**

In recent years, *in vitro* synthesis have been considered as an alternative to the conventional recombinant DNA technology, because of relatively high level of nucleic acid and protein production free from disadvantages associated with cellular expression. In vivo protein expression, being a powerful tool, has a set of significant limitations : (1) proteins produced *in vivo* could be subjected to degradation or post-translational modifications during cellular expression, such as glycosylation, deamination or oxidation; (2) cytotoxic or insoluble proteins may affect their synthesis and inhibit metabolic processes and the viability of the cell. To overcome these problems *in vitro* protein synthesis is mainly applied to (1) expression of toxic proteins; (2) radiolabeling of newly synthesized proteins and incorporation of unnatural amino acids; (3) screening quickly and economically for pharmaceuticals, food, environmental and commodity products.

The synthesis of proteins and other biological macromolecules in a cell-free system is based on cell extract. A typical *in vitro* cell-free system comprises : (1) a crude cell extract which contains the enzymes and factors necessary for transcription and translation [Zubay G: In vitro synthesis of proteins in microbial system, Annu Rev Genet 1973, 7: 267-287], (2) NTPs, (3) an energy regeneration system like acetyl phosphate (AcPh) and acetate kinase, phosphoenolpyruvate (PEP) and pyruvate kinase or creatine phosphate (CrPh) and creatine kinase, and (4) DNA and RNA.

However so called batch mode of cell-free gene expression systems has not been widely accepted as a practical alternative due to the short time of protein synthesis. After the development of a continuous flow cell-free (CFCF) system by Spirin et al. (Science 242: 1162-1164, 1988), where reaction time could be extended up to 50 hours, several improvements have been done by others (Kim DM, Choi CY: A semicontinous prokaryotic coupled cell-free system using a dialysis membrane, Biotechnol Prog 1996, 12: 645-649; Madin K., Sawasaki T., Ogasawara T., Endo E: A highly efficient and robust cell-free protein synthesis system prepared from wheat embryos: plants apparently contain a suicide system directed at ribosomes, Proc Natl Acad Sci 2000, 97: 559-564).

Summaries of various applications demonstrated that at the moment both types of *in vitro* expression systems, batch mode system [Kawarasaki Y, Nakano H, Yamane T, Anal Biochem 1995, 226: 320-324 ; Nakano H, Tananka T, Kawarasaki Y, Yamane T, Biosci Biotechnol Biochem 1994, 58: 631-634 ; Kim DM, Kigawa T, Chen CY, Yokoyama S, Eur J Biochem 1996, 239: 881-886; Patnaik R, Swartz JR, BioTechniques 1998, 24: 862-868] and systems of continuous actions [Tulin EE, Ken-Ichi T, Shin-Ichiro E, Biotechnol Bioeng 1995, 45: 511-516 ; Kim DM, Choi CY, Biotechnol Prog 1996, 12: 645-649 ; Madin K., Sawasaki T., Ogasawara T., Endo E, Proc Natl Acad Sci 2000, 97: 559-564] are under intensive investigations and developing very fast.

The published and commercially available batch *in vitro* systems are able to synthesized up to 0.5 mg/ml, and the continuous reactors can produce up to 3-5 mg of desired proteins per ml of reaction. In case of wheat germ cell-free continuous expression system the reaction time was extended up to 5 days [Madin K., Sawasaki T., Ogasawara T., Endo E, Proc Natl Acad Sci 2000, 97: 559-564]. In these systems, the continuous removal of the inhibitory by-product as well as the continuous supply of substrates and energy source enable the continuous reaction system to support protein synthesis over long reaction periods.

The biochemical energy in a cell-free system is supplied by the hydrolysis of triphosphates. For efficient transcription and translation the triphosphate concentration is maintained by an energy regeneration system: creatine phosphate with creatine phosphokinase, phosphoenolpyruvate with pyruvate kinase, and acetyl phosphate with endogenous *E. coli* acetate kinase [Ryabova LA, Vinokurov LM, Shekhovtsava EA, Alakhov YB, Spirin AS, Anal Biochem 1995, 226: 184-186].

Studies of triphosphate levels in different cell-free systems detected a high rate of their hydrolysis, to inorganic phosphate and diphosphates, mainly independently of protein synthesis and these correlates with sudden cessation of protein synthesis [Yao SL, Shen XC, Suzuki E, J Ferment Bioeng 1997, 84:7-13 ; Kim RG, Choi CY, J Biotechnol 2000, 84: 27-32]. The inhibitory effect of accumulating phosphate was also shown directly [Kim D-M, Swartz JR, Biotechnol Prog 2000, 16: 385-390].

It was proposed before that accumulation of inorganic phosphate during pyrophosphorolysis could also limit sequencing process and amplification of nucleic acids (WO9822615). WO9822615 describes methods for preventing inhibition of nucleic acid synthesis by pyrophosphate, where pyrophophorolysis is avoided by adding an enzyme such as a pentosyltransferase, a nucleotidyltransferase or a carboxy-lyase to the reaction, leading to the removal of pyrophosphate.

Significant modification of ATP-regeneration system for *E. coli in vitro* expression was made by Swartz [Kim D-M, Swartz JR, Biotechnol and Bioengin 1999, **66**: 180-188; Kim D-M et al, Biotechnol and Bioengin 2001, 74, 309-316; US patent 6,168,931; US patent 6,337,191] where standard energy sources like acetyl phosphate, phosphoenolpyruvate (PEP) and pyruvate kinase (PK) have been replaced by pyruvate in combination with the enzyme pyruvate oxidase to generate acetyl phosphate. The use of glycolytic intermediates or glucose energy source in combination with NADH and NAD$^+$ was also proposed. Thus, energy source like PEP that also increases the net free phosphate in the reaction mix was omitted. However, the use of glycolytic intermediates, glucose or pyruvate generally support protein synthesis for a period of time no longer than two hours, indicating decrease of NTP's regeneration efficiency or net accumulation of free phosphate.

As shown from the above, the low production protein amount is a main drawback of industrialization of cell-free protein synthesis. Therefore, improvements are still required in terms of total productivity of the protein by increasing the specific production rate and the length of system operation by regenerating an expensive energy source in order to both recycle the regenerated energy source and consume inorganic phosphate which is a strong protein synthesis inhibitor.

### Definitions.

In vitro transcription-translation and cell-free transcription-translation refer to any method for cell-free synthesis of a desired protein from DNA encoding the desired protein.

*In vitro* translation and cell-free translation refer to any method for cell-free synthesis of a desired protein from ribonucleic acid (RNA) encoding the desired protein.

Cell-free protein synthesis and *in vitro* protein synthesis refer to both *in vitro* transcription-translation and in vitro translation.

Cell-free extract as used herein denote any preparation comprising the components of a cell's protein synthesis machinery wherein such components are capable of expressing a nucleic acid encoding a desired protein. Thus, a cell free extract comprises components that are capable of translating messenger ribonucleic acid (mRNA) encoding a desired protein.

Biological macromolecules, biological materials and biological polymermolecules refer to i.e. nucleic acids, proteins and fragments thereof.

Cell-free extract enriched with ATP-sulfurylase or cell-free extract containing extra ATP-sulfurylase means that the ATP-sulfurylase concentration is beyond what is usual. The increased or extra amount of ATP-sulfurylase was obtained either by externally adding exogenous ATP-sulfurylase to the cell-free extract or by preparing cell-free extract from cells transformed with a vector over-expressing ATP-sulfurylase.

Cell-free system enriched with ATP-sulfurylase means that the ATP-sulfurylase concentration in the system is beyond what is usual. It comprises either cell-free extract enriched with ATP-sulfurylase and all components used for macromolecules synthesis or standard cell-free extract, all components used for macromolecules synthesis in which extra ATP-sulfurylase was added.

NTPs refer to nucleotide triphosphates (ATP, UTP, GTP and CTP).

### Summary of the invention.

The present invention provides methods for enhancing *in vitro* synthesis of proteins and fragments thereof in a cell-free system containing adenosine 5' phosphosulfate where ATP is required as a primary energy source, wherein said cell-free system is enriched with ATP-sulfurylase. The invention relates also to cell-free system containing adenosine 5' phosphosulfate and components that are capable of translating messenger ribonucleic acid encoding a desired protein enriched with ATP-sulfurylase and cell free extract containing adenosine 5' phosphosulfate and components that are capable of translating messenger ribonucleic acid encoding a desired protein enriched with ATP-sulfurylase. These biological macromolecules can have various origins (Procaryotic and Eucaryotic).

The enhancing *in vitro* synthesis methods and compositions provided by the invention are useful for *in vitro* production of proteins and fragments thereof.

ATP-sulfurylase plays several different roles in nature. In fungi, yeasts, most heterotrophic bacteria, algae, and higher plants, ATP-sulfurylase catalyzes the first intracellular reactions in the assimilation of sulfate into reduced organic molecules. In anaerobic sulfate reducing bacteria, ATP sulfurylase forms APS (adenosine 5'-phosphosulfate) solely to serve as the terminal electron acceptor of heterotrophic metabolism. In certain chemo- and photolithotrophic bacteria, ATP sulfurylase catalyzes the last reaction in the oxidation of reduced inorganic sulfur compounds to sulfate. In sulfate-assimilating organism, ATP sulfurylase (ATP: sulfate adenyltransferase, EC 2.7.7.4) catalyzes the first intracellular reaction in the incorporation of inorganic sulfate ($SO_4^{2-}$) into organic molecules, such as adenosine 5'-phosphosulfate (APS) [Karamohamed S et al.: Production, purification, and luminometric analysis of recombinant *saccharomyces cerevisiae* MET3 adenosine triphosphate sulfurylase expressed in *Escherichia coli,* Protein expression and Purification 1999, 15: 381-388].

ATP sulfurylase is widely distributed in nature and has been purified from a number of sources including lower and higher microorganisms, plants and animals. ATP sulfurylase genes have been also cloned from procaryotes, lower eucaryotes, plants, as well as animals.

ATP sulfurylase has been used for an application relating to DNA sequencing (WO9813523A1, Nyren et al. Anal. Biochem., (1985) 151, 504-509). The method is based on the detection of base incorporation by release of pyrophosphate (PPi). In the assay, the PPi which is generated is subsequently converted to ATP by ATP sulfurylase and the ATP production is monitored by luciferase. However, in the assay, ATP production is not used for DNA synthesis but only for the detection process.

ATP-sulfurylase utilizes inorganic phosphate for regeneration of ATP as shown in the scheme hereunder, which is the most commonly used source of energy but the other three NTPs are also used in protein expression and ATP can be used for their regeneration in a cell extract.

$$PPi + APS \quad \xleftarrow{\quad \text{ATP-sulfurylase} \quad} \quad ATP + SO_4^{2-}$$

## Description of the figures.

Figure 1 shows the synthesis of β-lactamase versus ATP-sulfurylase concentration in an *E. coli* cell-free transcription-translation system from Invitrogen.

Figure 2 shows the synthesis of β-lactamase versus ATP-sulfurylase concentration in an *E. coli* cell-free transcription-translation system from Novagen.

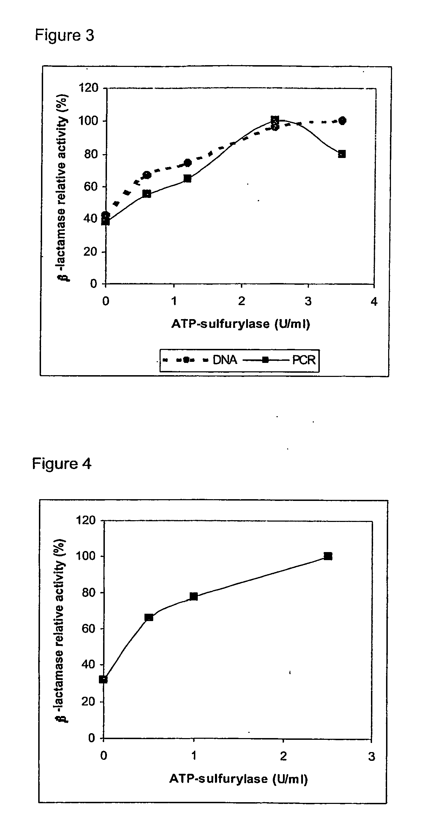

Figure 3 shows the synthesis of β-lactamase versus ATP-sulfurylase concentration in a cell-free transcription-translation system based on the *E. coli* S30 extract constructed mainly according to Kigawa et al (1999)

Figure 4 shows the synthesis of β-lactamase versus ATP-sulfurylase concentration in a cell-free translation system based on the *E. coli* S30 extract constructed mainly according to Kigawa et al (1999)

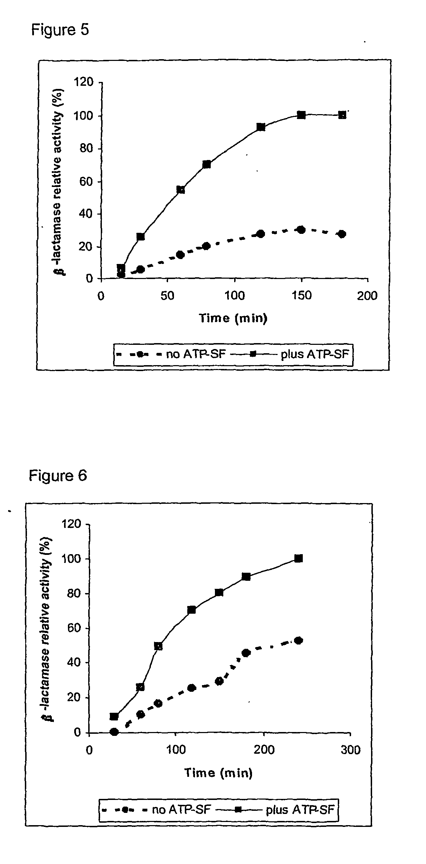

Figure 5 shows the kinetics of synthesis of β-lactamase in the absence or presence of ATP-sulfurylase in a cell-free translation system based on the *E. coli* S30 extract.

Figure 6 shows kinetics of the synthesis of β-lactamase in the absence or presence of ATP-sulfurylase in a cell-free transcription-translation system based on the *E. coli* S30 extract.

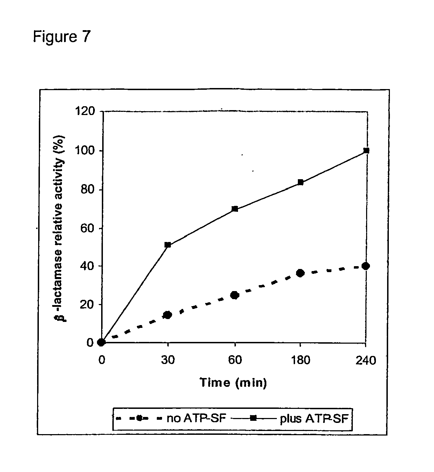

Figure 7 shows the synthesis of β-lactamase in the absence or presence of ATP-sulfurylase in a Bacillus subtilis cell-free translation system.

## Detailed description.

Methods and compositions are provided for enhancing *in vitro* synthesis of proteins and fragments thereof in a cell-free system where ATP is required as a primary energy source. The method comprises synthesizing biological materials in a reaction utilizing NTPs as a primary energy source, wherein NTPs are regenerated using energy regeneration system.

The method according to the present invention is characterized in that the *in vitro* synthesis of biological macromolecules is performed with a cell-free system enriched with ATP-sulfurylase and containing adenosine 5' phosphosulfate where ATP is required as primary energy source.

ATP-sulfurylase can regenerate ATP by utilization of APS and inorganic phosphate removing inorganic phosphate which causes inhibition of protein synthesis. When ATP-sulfurylase was added to a transcription-translation cell-free system alone or with an appropriate concentration of APS, it stimulated protein synthesis for a significant yield. While not limiting to the subject matter of the invention, at least two mechanisms may be proposed for the action of ATP-sulfurylase in a cell-free systems : (1) ATP regeneration and (2) utilization of inorganic phosphate.

Therefore, the cell free reaction system may comprise extra ATP which is regenerated using the cell-free extract enriched with ATP-sulfurylase.

In an other embodiment, the cell free reaction system may comprise exogenous APS.

The *in vitro* synthesis according to the method of the invention relates more particularly to translation of mRNA to produce polypeptides eventually after a transcription of said mRNA from a DNA template. Therefore, the method of the invention further relates to a coupled transcription-translation *in vitro* synthesis. In these embodiments, the cell free system contains all substances necessary for the transcription of mRNA from a DNA template and translation of said mRNA.

The *in vitro* synthesis according to the method of the invention can be carried out in a reaction vessel as a batch reaction.

The *in vitro* synthesis according to the method of the invention can also be carried out continuously or semi-continuously.

Extra ATP-sulfurylase of the cell-free system enriched with ATP-sulfurylase according to the present invention can be derived from prokaryotic organism, eukaryotic organism, transgenic vector, bacterial cell that has been genetically modified, *E. coli* extract, or can be purified. The extract can be prepared from cells transformed with a vector expressing ATP-sulfurylase.

In a first embodiment of the invention, ATP-sulfurylase is added to the cell-free system in order to obtain a cell-free system enriched with ATP-sulfurylase (extra ATP-sulfurylase). More particularly, extra ATP-sulfurylase is added to the cell-free extract.

Extra ATP-sulfurylase can be added to the cell-free system at the beginning and/or during the *in vitro* synthesis. ATP-sulfurylase can be added at intervals during the synthesis.

Extra ATP-sulfurylase can be present in the cell-free system prior to the beginning of the *in vitro* synthesis reaction. For example, extra ATP-sulfurylase can be present in the cell-free extract.

The invention describes also the cell-free system comprising a cell-free extract prepared from cells transformed with a vector over-expressing ATP-sulfurylase.

In practice, ATP-sulfurylase concentration is adapted according to experimental conditions and particularly to the protein to be expressed.

Optimization of ATP-sulfurylase concentration required for an optimal protein expression in a cell-free system can be achieved by titration of ATP-sulfurylase in a range for example from 0.1 to 10 U/ml with or without APS. The concentrations which support maximum of protein production and longer duration time of protein expression in a linear mode can be considered as an optimized for these experimental conditions.

Advantageously ATP-sulfurylase is present in the cell free extract enriched with ATP-sulfurylase at an initial concentration of at least about 0.1 U/ml and more advantageously of about 0.5 U/ml

The present invention relates also to a cell-free system containing adenosine 5' phosphosulfate and components that are capable of translating messenger ribonucleic acid encoding a desired protein enriched with ATP-sulfurylase.

A cell free system enriched with ATP-sulfurylase according to the present invention constitutes a cell free reaction mix for *in vitro* synthesis of proteins and fragments thereof where ATP is required as a primary energy source. It can be prepared from any commercial cell-free reaction mix such as Novagen or Invitrogen, or standard or modified S30 extract by adding extra ATP- sulfurylase to said cell free reaction mix. Therefore, the invention is directed to cell-free system comprising a cell-free extract enriched with ATP-sulfurylase.

The cell-free system can further comprise exogenous APS. It can also comprise all substances necessary for the translation of mRNA and transcription of mRNA from a DNA template.

As indicated above, extra ATP-sulfurylase presents in the cell free extract enriched with ATP-sulfurylase of the invention can be derived from prokaryotic organism, eukaryotic organism, transgenic vector, bacterial cell that has been genetically modified, *E. coli* extract, or can be purified.

The cell-free extract enriched with ATP-sulfurylase can be prepared from cells transformed with a vector over-expressing ATP-sulfurylase.

Advantageously, ATP-sulfurylase is present in a concentration of at least about 0.1 U/ml and more preferentially at least about 0.5 U/ml.

An example of a cell-free system on the basis of which can be prepared a cell-free system enriched with ATP-sulfurylase according to the present invention comprises :

- cell-free extract which contains the enzymes and factors necessary for transcription and translation;
- monomers for the macromolecule to be synthesized (e.g. amino acids),
- an energy regeneration system like acetyl phosphate (AcPh) and acetate kinase, phosphoenolpyruvate (PEP) and pyruvate kinase, or creatine phosphate (CrPh) and creatine kinase, etc., and
- eventually a template for production of macromolecule, e.g. DNA and/or mRNA etc.

Such cell-free systems are well-known in the art, and have been described in the literature (see for example Kigawa et al., 1999).

In the invention the cell-free system is further enriched with ATP-sulfurylase.

The invention also relates to a cell-free extract containing adenosine 5' phosphosulfate and components that are capable of translating messenger ribonucleic acid encoding a desired protein enriched with ATP-sulfurylase according to the above cell-free system.

An example of a cell-free extract on the basis of which can be prepared a cell-free extract enriched with ATP-sulfurylase according to the present invention comprises the enzymes, factors and components of a cell's protein synthesis machinery e.g. ribosomes, tRNA, polymerases, transcriptional factors, etc.

Such cell-free extracts are well-known in the art, and have been described in the literature (Zubay G et al. (1973) In vitro synthesis of proteins in microbial system, Annu Rev Genet, 7:267287 ; Roberts BE et al. (1973) Efficient translation of tobacco mosaic virus RNA and rabbit globin 9S RNA in a cell-free system from commercial wheat germ, Proc Natl Acad Sci USA, 70: 2330-2334 ; Pelham HRB et al. (1976) An efficient mRNA-dependent translation system from reticulocyte lysates, Eur J Biochem, 67:247-256 ; Shimizu Y. et al (2001) Cell-free translation reconstituted with purified components, Biotech. Nature, 19, 751-755).

**Examples.**

The following examples are offered to illustrate but not to limit the present invention.

Example 1 Synthesis of β-lactamase versus ATP-sulfurylase concentration in a cell-free transcription-translation system from Invitrogen.

The standard reaction mixture of 50 $\mu$l for cell-free transcription-translation system employing the ATP-sulfurylase-dependent utilization of inorganic phosphate and ATP regeneration consist of the following components in 50 $\mu$l : 25 $\mu$l of IVPS *E. coli* Extract (composition proprietary of Invitrogen, Expressway *in vitro* protein synthesis system *E. coli*. Cat n°. 12333.019), 20 $\mu$l of 2.5xIVPS *E. coli* reaction buffer (composition proprietary of Invitrogen), 1 $\mu$l of T7 RNA polymerase (composition proprietary of Invitrogen), and 0.1 mg/ml of PCR product encoding for β-lactamase. In this reaction, ATP-sulfurylase was added in concentrations of 1.2 and 2.5 U/ml. Reactions were conducted for 2 h in a water bath set at 37°C according to manufacturer's instructions (Invitrogen). Detection of β-lactamase activity was done by nitrocefin test as described by O'Callaghan et al. [O'Callaghan C.H., Morris S.M., Kirby S.M., Shingler A.H, Antimicrob. Agents Chemother 1972, 1: 283-288] . 50 $\mu$l from cell-free transcription-translation reaction mix, diluted 600 fold, was mixed with 150 $\mu$l of nitrocefin (16 $\mu$g/ml) in a well of a microtitration plate and OD at 486 nm was measured. The highest value of β-lactamase expression is set to 100%. S. cerevisiae ATP-sulfurylase was from Sigma.
The use of exogenous ATP-sulfurylase seems to be significant in the expression of proteins particularly sensitive to the concentration of inorganic phosphate. The final yield of β-lactamase was at least 3 fold higher than that of the experiment carried out without added exogenous ATP-sulfurylase.

Example 2 : Synthesis of β-lactamase versus ATP-sulfurylase concentration in an *E. coli* cell-free transcription-translation system from Novagen.

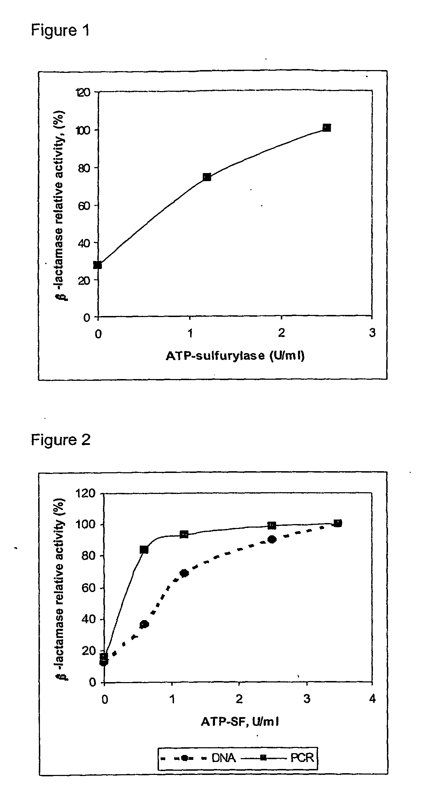

The standard reaction mixture of 50 $\mu$l for cell-free transcription-translation system employing the ATP-sulfurylase-dependent utilization of inorganic phosphate and ATP regeneration consist of the following components in 50 $\mu$l : 35 $\mu$l of EcoPro Reaction Mix (composition proprietary of Novagen, Introductory EcoPro™ T7 System. Cat. N°. 70888-3), 2 $\mu$l of 5 mM Methionine, and 0.1 mg/ml of PCR product or 0.1 mg/ml of plasmid DNA, both encoding for β-lactamase.
In this commercial extract ATP-sulfurylase was added in concentrations of 0.6, 1.2, 2.5 and 3.5 U/ml. Reactions were conducted for 1 h in a water bath set at 37°C according to manufacture instructions (Novagen). Detection of β-lactamase activity was done by nitrocefin test (see Fig. 1). The highest value of β-lactamase expression for PCR or DNA template is set to 100%. 50 $\mu$l from the cell-free transcription-translation reaction mix, diluted 25 fold, was mixed with 150 $\mu$l of nitrocefin (16 ($\mu$g/ml) in a well of a microtitration plate and OD at 486 nm was measured. S. cerevisiae ATP-sulfurylase was from Sigma.
The effect of added ATP-sulfurylase on the β-lactamase expression level is striking, reaching at least 6-fold higher level than that without ATP-sulfurylase in the *E. coli* cell-free transcription-translation system from Novagen.

Example 3 : Synthesis of β-lactamase in the absence or presence of ATP-sulfurylase in a cell-free transcription-translation system constructed according Kigawa et al.

The standard reaction mixture of 50 $\mu$l for cell-free transcription-translation system as described by Kigawa (Kigawa T., Yabuki T., Yoshida Y., Tsutsui M., Ito Y., Shibata T., Yokoyama S: Cell-free production and stable-isotope labeling of milligram quantities of proteins, FEBS Lett. 1999, 442: 15-19]) where creatine phosphate and creatine phosphokinase were replaced by 60 mM of acetyl phosphate. The transcription-translation reactions were carried out with 0.1 mg/ml of PCR product or 0.1 mg/ml of plasmid DNA encoding for β-lactamase. In the S30 extract ATP-sulfurylase was added in concentrations of 0.6, 1.2, 2,5 and 3.5 U/ml. Reactions were conducted for 3 h in a water bath set at 30°C. 50 $\mu$l from cell-free transcription-translation reaction mix, diluted 600 fold, was mixed with 150 $\mu$l of nitrocefin (16 $\mu$g/ml) in a well of a microtitration plate and OD at 486 nm was measured. The highest value of β-lactamase expression for PCR or DNA template is set to 100%.
S30 *E. coli* extract was prepared as described in Zubay. T7 RNA polymerase was from (Hybaid), S. cerevisiae

ATP-sulfurylase was from Sigma.

The ATP-sulfurylase addition into the *E. coli* cell-free transcription-translation system prepared as described by Kigawa et al (1999) increases the level of β-lactamase expression up to 2,5-fold, similar to the result with the Invitrogen mix.

Table 1 hereunder reports the stimulation of β-lactamase expression by addition of ATP-SF in different transcription-translation cell-free systems programmed with PCR templates.

Table 1

| | β-lactamase relative activity (%) in cell-free transcription-translation systems/ATP-SF, U/ml | | | | |
|---|---|---|---|---|---|
| | no | 0.6 | 1.2 | 2.5 | 3,5 |
| Invitrogen | 27,4 | | 74,2 | 100% | |
| Novagen | 15,6% | 83,8% | 93,1% | 98,7% | 100% |
| Kigawa et al | 38,2 | 55% | 64,5% | 100% | 80% |

Example 4 : Synthesis of β-lactamase in the absence or presence of ATP-sulfurylase in a cell-free translation system constructed similar to Kigawa et al.

The standard reaction mixture of 50 μl for cell-free translation system as described by Kigawa (1999) - with 12 mM Mg(OAc)$_2$ and 0,2 mg/ml mRNA was used. Creatine phosphate and creatine phosphokinase were replaced by 60 mM of acetyl phosphate. In the S30 extract ATP-sulfurylase was added in concentrations of 0.5, 1.0 and 2.5 U/ml. Reactions were conducted for 3 h in a water bath set at 30°C. 50 μl from cell-free transcription-translation reaction mix, diluted 600 fold, was mixed with 150 μl of nitrocefin (16 μg/ml) in a well of a microtitration plate and OD at 486 nm was measured. The highest value of β-lactamase expression is set to 100%.

S30 *E. coli* extract was prepared as described by Zubay. T7 RNA polymerase was from (Hybaid), *S. cerevisiae* ATP-sulfurylase was from Sigma.

The ATP-sulfurylase addition into the *E. coli* cell-free transcription-translation system prepared as described by Kigawa et al (1999) increases the level of β-lactamase expression up to 3-fold, similar to the result with the *E. coli* transcription-translation systems.

Example 5 : Kinetics of synthesis of β-lactamase in the absence or presence of ATP-sulfurylase in an *E. coli* cell-free translation system.

The standard reaction mixture of 50 μl for cell-free translation system as described by Kigawa (1999) consists of the following components in 50 μl: 60 mM Hepes pH 8.2, 10 mM Mg(OAc)2, 80 mM NH4(OAc), 1.2 mM ATP, 0.8 mM GTP, 1 mM DTT, 0.64 mM cAMP, 200 mM potassium glutamate, 60 mM acetyl phosphate, 34 μg/μl folinic acid, 0.2 mg/ml of mRNA, 1 U/μl T7 polymerase with 0.3 volumes of S30 was used.

In the S30 extract prepared as described by Zubay et al. (1973) ATP-sulfurylase was added in concentrations of 1.5 U/ml. Reactions were conducted in a water bath set at 30°C. 50 μl taken from cell-free transcription-translation reaction mix at different time points, diluted 600 fold, were mixed with 150 μl of nitrocefin (16 μg/ml) in a well of a microtitration plate and OD at 486 nm was measured. The highest value of β-lactamase expression is set to 100%.

S. cerevisiae ATP-sulfurylase was from Sigma.

Table 2 hereunder reports the β-lactamase relative activity (%)versus reaction time in the absence and presence of ATP-sulfurylase.

Table 2

| | β-lactamase relative activity (%) in *E. coli* cell-free translation system | |
|---|---|---|
| Reaction time (min) | Without ATP-SF | With ATP-SF |
| 15 | 1.8 | 6.3 |
| 30 | 5.4 | 26 |
| 60 | 14 | 54 |
| 80 | 20 | 70 |
| 120 | 27 | 92 |

(continued)

| | β-lactamase relative activity (%) in *E. coli* cell-free translation system | |
|---|---|---|
| Reaction time (min) | Without ATP-SF | With ATP-SF |
| 150 | 30 | 100 |
| 180 | 27 | 100 |

The kinetics of β-lactamase cell-free synthesis show that a 3-fold increase in protein production could be observed after 30 minutes of translation system incubation.

Example 6 : Synthesis of β-lactamase in the absence and presence of ATP-sulfurylase from *S. cerevisiae* in an *E. coli* cell-free transcription-translation system.

The standard reaction mixture of 50 μl for cell-free translation system as described by Kigawa (1999)employing the ATP-sulfurylase-dependent utilization of inorganic phosphate and ATP regeneration where creatine phosphate and creatine phosphokinase were replaced by 60 mM of acetyl phosphate. In the reaction mixture ATP-sulfurylase was added in concentrations of 1.5 U/ml. Reactions were conducted for 3 h in a water bath set at 30°C. 50 μl aliquotes were taken from cell-free transcription-translation reaction mix at different time points, diluted 600 fold an then were mixed with 150 μl of nitrocefin (16 μg/ml) in a well of a microtitration plate and OD at 486 nm was measured. The highest value of β-lactamase expression is set to 100%.

T7 RNA polymerase was from (Hybaid), S. cerevisiae ATP-sulfurylase was from Sigma.

Table 3 hereunder reports the β-lactamase relative activity (%) versus reaction time in the absence and presence of ATP-sulfurylase.

Table 3

| | β-lactamase relative activity (%) in *E. coli* cell-free transcription-translation system | |
|---|---|---|
| Reaction time (min) | Without ATP-SF | With ATP-SF |
| 30 | 4.7 | 8.6 |
| 60 | 10 | 26 |
| 80 | 16.2 | 49 |
| 120 | 25 | 70 |
| 150 | 29 | 80 |
| 180 | 45 | 89 |
| 240 | 52 | 100 |

These experiments show that ATP-sulfurylase has an effect on β-lactamase synthesis. Indeed, β-lactamase production after one hour of reaction was at least two fold greater than the one obtained without ATP-sulfurylase.

Example 7 : Synthesis of β-lactamase with and without ATP-sulfurylase in a *Bacillus subtilis* cell-free translation system.

The standard reaction mixture of 50 μl for cell-free translation system employing the ATP-sulfurylase-dependent utilization of inorganic phosphate and ATP regeneration as mainly described by Kigawa (1999) with 0.3 volumes of S30 extract prepared as described by Chambliss et al. (1983). Modified cell-free translation system consists of 10 mM Mg (OAc)2, 50 mM acetyl phosphate and 0,2 mg/ml mRNA. In the S30 extract ATP-sulfurylase was added in concentrations of 1 U/ml. Reactions were conducted for 3 h in a water bath set at 30°C. 50 μl aliquots were taken from cell-free transcription-translation reaction mix at different time points, diluted 500 fold, were mixed with 150 μl of nitrocefin (16 μg/ml) in a well of a microtitration plate and OD at 486 nm was measured. The highest value of β-lactamase expression is set to 100%.

S. cerevisiae ATP-sulfurylase was from Sigma.

Table 4 hereunder reports the β-lactamase relative activity (%) in the cell-free translation system based on Bacillus subtilis extract in the absence and presence of ATP sulfurylase

Table 4

| Time (min) | Cell-free translation without ATP-SF | Cell-free translation with ATP-SF |
|---|---|---|
| 30 | 14,4 | 51 |
| 60 | 24,5 | 69,4 |
| 180 | 36,1 | 83 |
| 240 | 40,4 | 100 |

The production of β-lactamase in the *Bacillus subtilis* transcription-translation system in the presence of ATP-sulfurylase was increased up to 2.5 fold compared to the one obtained without ATP-sulfurylase. The effect was observed after 30 minutes of incubation.

**Claims**

1. A method for enhancing *in vitro* synthesis of proteins and fragments thereof in a cell-free system containing adenosine 5' phosphosulfate where ATP is required as a primary energy source, wherein said cell-free system is enriched with ATP-sulfurylase

2. A method according to claim 1, wherein the cell free system comprises exogenous adenosine 5' phosphosulfate.

3. The method according to anyone of claims 1 or 2, wherein said *in vitro* synthesis comprises also transcription of mRNA from a DNA template.

4. A method according to anyone of claims 1 to 3, wherein said *in vitro* synthesis is carried out in a reaction vessel as a batch reaction, semi continuously or continuously.

5. A method according to anyone of claims 1 to 4, wherein ATP-sulfurylase is added to the cell-free system at the beginning and/or during the *in vitro* synthesis or at intervals during the *in vitro* synthesis.

6. A method according to anyone of claims 1 to 5, wherein the cell-free system comprises a cell-free extract prepared from cells transformed with a vector over-expressing ATP-sulfurylase.

7. A method according to anyone of claims 1 to 6, wherein ATP-sulfurylase concentration is adapted according to the experimental conditions and the biological macromolecules to be synthesized.

8. A method according to anyone of claims 1 to 7, wherein ATP-sulfurylase is present in the cell-free system at an initial concentration of at least 0.1 U/ml.

9. A cell-free system containing adenosine 5' phosphosulfate and components that are capable of translating messenger ribonucleic acid encoding a desired protein enriched with ATP-sulfurylase.

10. A cell-free system according to claim 9 comprising exogenous adenosine 5' phosphosulfate.

11. A cell-free system according to anyone of claims 9 or 10 comprising all substances necessary for the translation of mRNA and transcription of mRNA from a DNA template.

12. A cell-free system according to anyone of claims 9 to 11, wherein extra ATP-sulfurylase is derived from prokaryotic organism, eukaryotic organism, transgenic vector, bacterial cell that has been genetically modified, *E. coli* extract, or is purified.

13. A cell-free system according to anyone of claims 9 to 12, wherein the cell-free extract enriched with ATP-sulfurylase is prepared from cells transformed with a vector over-expressing ATP-sulfurylase.

14. A cell-free system according to anyone of claims 9 to 13, wherein ATP-sulfurylase is present in a concentration of at least 0.1 U/ml.

15. A cell-free extract containing adenosine 5' phosphosulfate and components that are capable of translating messenger ribonucleic acid encoding a desired protein enriched with ATP-sulfurylase.

16. A cell-free extract according to claim 15 comprising exogenous adenosine 5' phosphosulfate.

17. A cell-free extract according to anyone of claims 15 or 16 comprising all substances necessary for the translation of mRNA and transcription of mRNA from a DNA template.

18. A cell-free extract according to anyone of claims 15 to 17, wherein extra ATP-sulfurylase is derived from prokaryotic organism, eukaryotic organism, transgenic vector, bacterial cell that has been genetically modified, *E. coli* extract, or is purified.

19. A cell-free extract according to anyone of claims 15 to 18 prepared from cells transformed with a vector over-expressing ATP-sulfurylase.

20. A cell-free extract according to anyone of claims 15 to 19, wherein ATP-sulfurylase is present in a concentration of at least 0.1 U/ml.

**Patentansprüche**

1. Methode zur Verbesserung der In-vitro-Synthese von Proteinen und Fragmenten davon in einem zellfreien System, das Adenosin-5'-phosphosulfat enthält, wenn ATP als primäre Energiequelle benötigt wird, wobei das zellfreie System mit ATP-Sulfurylase angereichert ist.

2. Methode nach Anspruch 1, wobei das zellfreie System exogenes Adenosin-5'-phosphosulfat umfasst.

3. Methode nach einem der Ansprüche 1 oder 2, wobei die In-vitro-Synthese auch die Transkription von mRNA von einer DNA-Matrize umfasst.

4. Methode nach einem der Ansprüche 1 bis 3, wobei die In-vitro-Synthese in einem Reaktionsgefäß als Batch-Reaktion, semikontinuierlich oder kontinuierlich durchgeführt wird.

5. Methode nach einem der Ansprüche 1 bis 4, wobei ATP-Sulfurylase dem zellfreien System zu Beginn und/oder während der In-vitro-Synthese oder in Abständen während der In-vitro-Synthese zugeführt wird.

6. Methode nach einem der Ansprüche 1 bis 5, wobei das zellfreie System einen aus mit einem ATP-Sulfurylase überexprimierenden Vektor transformierten Zellen hergestellten zellfreien Extrakt umfasst.

7. Methode nach einem der Ansprüche 1 bis 6, wobei die ATP-Sulfurylase-Konzentration gemäß den experimentellen Bedingungen und den zu synthetisierenden biologischen Makromolekülen angepasst wird.

8. Methode nach einem der Ansprüche 1 bis 7, wobei ATP-Sulfurylase im zellfreien System in einer Anfangskonzentration von mindestens 0,1 U/ml vorliegt.

9. Zellfreies System, enthaltend Adenosin-5'-phosphosulfat und Komponenten mit der Fähigkeit zur Translation von Botenribonukleinsäure, codierend für ein gewünschtes Protein, angereichert mit ATP-Sulfurylase.

10. Zellfreies System nach Anspruch 9, umfassend exogenes Adenosin-5'-phosphosulfat.

11. Zellfreies System nach einem der Ansprüche 9 oder 10, umfassend alle für die Translation von mRNA und Transkription von mRNA von einer DNA-Matrize notwendigen Substanzen.

12. Zellfreies System nach einem der Ansprüche 9 bis 11, wobei zusätzliche ATP-Sulfurylase aus einem prokaryontischen Organismus, eukaryontischen Organismus, transgenen Vektor, einer gentechnisch veränderten Bakterienzelle, einem E.-coli-Extrakt stammt oder aufgereinigt wird.

**13.** Zellfreies System nach einem der Ansprüche 9 bis 12, wobei der mit ATP-Sulfurylase angereicherte zellfreie Extrakt aus mit einem ATP-Sulfurylase überexprimierenden Vektor transformierten Zellen hergestellt wird.

**14.** Zellfreies System nach einem der Ansprüche 9 bis 13, wobei ATP-Sulfurylase in einer Konzentration von mindestens 0,1 U/ml vorliegt.

**15.** Zellfreier Extrakt, enthaltend Adenosin-5'-phosphosulfat und Komponenten mit der Fähigkeit zur Translation von Botenribonukleinsäure, codierend für ein gewünschtes Protein, angereichert mit ATP-Sulfurylase.

**16.** Zellfreier Extrakt nach Anspruch 15, umfassend exogenes Adenosin-5'-phosphosulfat.

**17.** Zellfreier Extrakt nach einem der Ansprüche 15 oder 16, umfassend alle für die Translation von mRNA und Transkription von mRNA von einer DNA-Matrize notwendigen Substanzen.

**18.** Zellfreier Extrakt nach einem der Ansprüche 15 bis 17, wobei zusätzliche ATP-Sulfurylase aus einem prokaryontischen Organismus, eukaryontischen Organismus, transgenen Vektor, einer gentechnisch veränderten Bakterienzelle, einem E.-coli-Extrakt stammt oder aufgereinigt wird.

**19.** Zellfreier Extrakt nach einem der Ansprüche 15 bis 18, hergestellt aus mit einem ATP-Sulfurylase überexprimierenden Vektor transformierten Zellen.

**20.** Zellfreies System nach einem der Ansprüche 15 bis 19, wobei ATP-Sulfurylase in einer Konzentration von mindestens 0,1 U/ml vorliegt.

## Revendications

**1.** Procédé destiné à amplifier la synthèse *in vitro* de protéines et de fragments de celles-ci dans un système dépourvu de cellules contenant de l'adénosine 5' phosphosulfate, dans lequel de l'ATP est requis en tant que source primaire d'énergie, dans lequel ledit système dépourvu de cellules est enrichi en ATP sulfurylase.

**2.** Procédé selon la revendication 1, dans lequel le système dépourvu de cellules comprend de l'adénosine 5' phosphosulfate exogène.

**3.** Procédé selon l'une quelconque des revendications 1 ou 2, dans lequel ladite synthèse *in vitro* comprend également la transcription d'ARNm à partir d'une matrice d'ADN.

**4.** Procédé selon l'une quelconque des revendications 1 à 3, dans lequel ladite synthèse *in vitro* est effectuée dans un récipient de réaction sous forme de réaction par lots, de manière semi-continue ou continue.

**5.** Procédé selon l'une quelconque des revendications 1 à 4, dans lequel une ATP sulfurylase est ajoutée au système dépourvu de cellules au début de la synthèse *in vitro* et/ou pendant celle-ci ou bien à des intervalles pendant la synthèse *in vitro.*

**6.** Procédé selon l'une quelconque des revendications 1 à 5, dans lequel le système dépourvu de cellules comprend un extrait dépourvu de cellules préparé à partir de cellules transformées avec un vecteur sur-exprimant une ATP sulfurylase.

**7.** Procédé selon l'une quelconque des revendications 1 à 6, dans lequel la concentration en ATP sulfurylase est adaptée en fonction des conditions expérimentales et des macromolécules biologiques devant être synthétisées.

**8.** Procédé selon l'une quelconque des revendications 1 à 7, dans lequel une ATP sulfurylase est présente dans le système dépourvu de cellules avec une concentration initiale d'au moins 0,1 U/ml.

**9.** Système dépourvu de cellules contenant de l'adénosine 5' phosphosulfate et des composants qui sont capables de traduire de l'acide ribonucléique messager codant pour une protéine souhaitée enrichie en ATP sulfurylase.

**10.** Système dépourvu de cellules, selon la revendication 9, comprenant de l'adénosine 5' phosphosulfate exogène.

**11.** Système dépourvu de cellules, selon l'une quelconque des revendications 9 ou 10, comprenant toutes les substances nécessaires à la traduction d'un ARNm et la transcription d'ARNm à partir d'une matrice d'ADN.

**12.** Système dépourvu de cellules selon l'une quelconque des revendications 9 à 11, dans lequel une ATP sulfurylase supplémentaire est dérivée à partir d'un organisme procaryote, d'un organisme eucaryote, d'un vecteur transgénique, d'une cellule bactérienne qui a été génétiquement modifiée, d'un extrait d'*E. coli*, ou bien est purifiée.

**13.** Système dépourvu de cellules selon l'une quelconque des revendications 9 à 12, dans lequel l'extrait dépourvu de cellules enrichi en ATP sulfurylase est préparé à partir de cellules transformées avec un vecteur sur-exprimant une ATP sulfurylase.

**14.** Système dépourvu de cellules selon l'une quelconque des revendications 9 à 13, dans lequel une ATP sulfurylase est présente en une concentration d'au moins 0,1 U/ml.

**15.** Extrait dépourvu de cellules contenant de l'adénosine 5' phosphosulfate et des composants qui sont capables de traduire de l'acide ribonucléique messager codant pour une protéine souhaitée enrichie en ATP sulfurylase.

**16.** Extrait dépourvu de cellules, selon la revendication 15, comprenant de l'adénosine 5' phosphosulfate exogène.

**17.** Extrait dépourvu de cellules, selon l'une quelconque des revendications 15 ou 16, comprenant toutes les substances nécessaires à la traduction d'un ARNm et la transcription d'ARNm à partir d'une matrice d'ADN.

**18.** Extrait dépourvu de cellules, selon l'une quelconque des revendications 15 à 17, dans lequel une ATP sulfurylase supplémentaire est dérivée à partir d'un organisme procaryote, d'un organisme eucaryote, d'un vecteur transgénique, d'une cellule bactérienne qui a été génétiquement modifiée, d'un extrait d'*E. coli*, ou bien est purifiée.

**19.** Extrait dépourvu de cellules, selon l'une quelconque des revendications 15 à 18, préparé à partir de cellules transformées avec un vecteur sur-exprimant une ATP sulfurylase.

**20.** Extrait dépourvu de cellules, selon l'une quelconque des revendications 15 à 19, dans lequel une ATP sulfurylase est présente en une concentration d'au moins 0,1 U/ml.

Figure 1

Figure 2

Figure 3

Figure 4

Figure 5

Figure 6

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9822615 A
- US 6168931 B
- US 6337191 B
- WO 9813523 A1

### Non-patent literature cited in the description

- **Zubay G.** In vitro synthesis of proteins in microbial system. *Annu Rev Genet,* 1973, vol. 7, 267-287
- **Spirin et al.** *Science,* 1988, vol. 242, 1162-1164
- **Kim DM ; Choi CY.** A semicontinous prokaryotic coupled cell-free system using a dialysis membrane. *Biotechnol Prog,* 1996, vol. 12, 645-649
- **Madin K. ; Sawasaki T. ; Ogasawara T. ; Endo E.** A highly efficient and robust cell-free protein synthesis system prepared from wheat embryos: plants apparently contain a suicide system directed at ribosomes. *Proc Natl Acad Sci,* 2000, vol. 97, 559-564
- **Kawarasaki Y ; Nakano H ; Yamane T.** *Anal Biochem,* 1995, vol. 226, 320-324
- **Nakano H ; Tananka T ; Kawarasaki Y ; Yamane T.** *Biosci Biotechnol Biochem,* 1994, vol. 58, 631-634
- **Kim DM ; Kigawa T ; Chen CY ; Yokoyama S.** *Eur J Biochem,* 1996, vol. 239, 881-886
- **Patnaik R ; Swartz JR.** *BioTechniques,* 1998, vol. 24, 862-868
- **Tulin EE ; Ken-Ichi T ; Shin-Ichiro E.** *Biotechnol Bioeng,* 1995, vol. 45, 511-516
- **Kim DM ; Choi CY.** *Biotechnol Prog,* 1996, vol. 12, 645-649
- **Madin K. ; Sawasaki T. ; Ogasawara T. ; Endo E.** *Proc Natl Acad Sci,* 2000, vol. 97, 559-564
- **Ryabova LA ; Vinokurov LM ; Shekhovtsava EA ; Alakhov YB ; Spirin AS.** *Anal Biochem,* 1995, vol. 226, 184-186
- **Yao SL ; Shen XC ; Suzuki E.** *J Ferment Bioeng,* 1997, vol. 84, 7-13
- **Kim RG ; Choi CY.** *J Biotechnol,* 2000, vol. 84, 27-32
- **Kim D-M ; Swartz JR.** *Biotechnol Prog,* 2000, vol. 16, 385-390
- **Kim D-M ; Swartz JR.** *Biotechnol and Bioengin,* 1999, vol. 66, 180-188
- **Kim D-M et al.** *Biotechnol and Bioengin,* 2001, vol. 74, 309-316
- *Protein expression and Purification,* 1999, vol. 15, 381-388
- **Nyren et al.** *Anal. Biochem.,* 1985, vol. 151, 504-509
- **Zubay G et al.** In vitro synthesis of proteins in microbial system. *Annu Rev Genet,* 1973, vol. 7, 267287
- **Roberts BE et al.** Efficient translation of tobacco mosaic virus RNA and rabbit globin 9S RNA in a cell-free system from commercial wheat germ. *Proc Natl Acad Sci USA,* 1973, vol. 70, 2330-2334
- **Pelham HRB et al.** An efficient mRNA-dependent translation system from reticulocyte lysates. *Eur J Biochem,* 1976, vol. 67, 247-256
- **Shimizu Y. et al.** Cell-free translation reconstituted with purified components. *Biotech. Nature,* 2001, vol. 19, 751-755
- **O'Callaghan C.H. ; Morris S.M. ; Kirby S.M. ; Shingler A.H.** *Antimicrob. Agents Chemother,* 1972, vol. 1, 283-288
- **Kigawa T. ; Yabuki T. ; Yoshida Y. ; Tsutsui M. ; Ito Y. ; Shibata T. ; Yokoyama S.** Cell-free production and stable-isotope labeling of milligram quantities of proteins. *FEBS Lett.,* 1999, vol. 442, 15-19